# EUROPEAN PATENT SPECIFICATION

(11) **EP 2 547 255 B1**
(45) Date of publication and mention of the grant of the patent: **30.09.2015**
(21) Application number: 11712752.2
(22) Date of filing: 18.03.2011
(51) Int. Cl.: A61B 5/055, A61B 5/11, G01R 33/28, G02B 23/24

(54) **OPTICAL MOTION TRACKING OF AN OBJECT**
OPTISCHE BEWEGUNGSVERFOLGUNG EINES OBJEKTS
SUIVI OPTIQUE DES MOUVEMENTS D'UN OBJET

(30) Priority: 21.04.2010 US 326283 P; 18.03.2010 DK 201070106
(43) Date of publication of application: 23.01.2013
(73) Proprietor: Rigshospitalet, 2100 Copenhagen Ø (DK)
(72) Inventor: OHLHUES, Anders, DK-1828 Frederiksberg C (DK); THOMSEN, Carsten, DK-2800 Lyngby (DK)
(74) Representative: Høiberg A/S
(86) International application number: PCT/DK2011/050089
(87) International publication number: WO 2011/113441

(56) References cited:
- WO-A1-98/38908
- US-A- 6 122 541
- US-A1- 2004 138 556
- US-A1- 2004 258 306
- US-A1- 2005 283 068
- US-A1- 2006 074 296
- US-B1- 6 724 930
- US-B2- 7 672 712
- QIN LEI ET AL: "Prospective head-movement correction for high-resolution MRI using an in-bore optical tracking system.", MAGNETIC RESONANCE IN MEDICINE : OFFICIAL JOURNAL OF THE SOCIETY OF MAGNETIC RESONANCE IN MEDICINE / SOCIETY OF MAGNETIC RESONANCE IN MEDICINE OCT 2009 LNKD- PUBMED:19526503, vol. 62, no. 4, 12 June 2009 (2009-06-12), XP002613247, ISSN: 1522-2594

## Description

The present invention relates to a system and a method for monitoring / tracking the movement of an object in a location which is difficult to access, such as the movement of a patient in a clinical MRI scanner. The invention further relates to the use of a borescope for motion tracking of an object and a marker plate suitable for use in a motion tracking system.

### Background of invention

Modern clinical / medical in vivo scanning techniques, such as Magnetic Resonance Imaging (MRI), are excellent for assessing the structure, physiology, chemistry and function of human organs, in particular the brain. Because the object of interest, i.e. the patient, is often imaged in many slices and scanning steps in order to build a complete three dimensional view, scans are of long duration, usually lasting several minutes. To increase resolution (detail) of a scan, more slices and more scanning steps must be used, which further increases the duration of a scan. Many clinical imaging techniques rely on detecting minute variations in a particular type of signal, which makes these techniques even more susceptible to patient movements.

For long duration scans motion of the patient is a substantial problem associated with the acquirement of accurate data. Consequently, patients are commonly required to lie still over extended time periods. Similar requirements exist for other modern imaging techniques, such as Positron Emission Tomography (PET), Single Photon Emission Computerized Tomography (SPECT) and Computer Tomography (CT). PET scans are increasingly combined with CT and/or MRI scans providing both anatomic and metabolic information. These co-registration techniques will in the future require further limitations to patient movement during scans.

Image guided therapy e.g. stereotactic radiotherapy, stereotactic radio surgery, focusing ultrasound health therapy and laser therapy, rely on keeping a fixed geometry during the duration of the therapy. Patient motion may result in misalignment of the target volume e.g. tumour and the externally applied radiation field.

Besides the problem of patient movement during scan or radiotherapy, the physical space in a scanner or a radiotherapy apparatus may be very limited and the patient may be located in a strong magnetic or radioactive field which can prevent use of electronic devices inside the scanning volume. Several techniques have been developed over the past decades to track the movement of the patient and to reduce the sensitivity of scans to motion of the patient. A review of prior art techniques may be found in the introductory part of WO 2007/136745. Pages 2 to 8 of WO 2007/136745 are therefore incorporated herein by reference.

WO 2007/136745 circumvents the limitations of the prior art techniques like stereovision systems where more than one camera is required, by teaching a solution where an object orientation marker is attached to the patient and imaged in a single camera by means of one or more mirrors attached to the camera. Movement of the patient in the scanner coordinate system can thereby be calculated.

Single camera tracking is also described in WO 2004/023783 wherein a plurality of markers attached to the patient is imaged in a single camera and converted to patient motion coordinates.

Single camera MRI motion tracking is disclosed in US 2005/0283068. A camera and an IR source are located outside the magnetic field and the bore of the scanner. Infrared reflectors located on the patient's head are illuminated by the IR source and imaged in the camera. The camera is enclosed in a Faraday cage and a tubular wave guide is provided as an RF cage around the IR source and the camera lens.

### Summary of invention

These single camera solutions are however by nature limited in resolution because due to the powerful electromagnetic field in the scanner bore the camera must be located outside the scanner bore whereas the markers are attached to the patient inside the narrow scanner bore which is difficulty accessible. One object of the invention is therefore to improve the known object tracking systems.

This is achieved by an optical motion tracking system for determining the movement of an object at least partly located in a volume of difficult access and/or at least partly located in an electromagnetic field, said system comprising:
- a borescope for imaging a pattern on the object or a surface part of the object with a camera, said pattern or surface part located adjacent to a distal end of the borescope and said camera attached to a proximal end of said borescope, and
- image processing means for calculating the movement of said pattern or surface part relative to the distal end of the borescope based on a plurality of frames / images captured by the camera.

The invention further relates to a method for determining the movement of an object at least partly surrounded by an electromagnetic field, said method comprising the steps of:
- imaging a pattern on the object or a surface part of the object in a camera, said pattern or surface part located adjacent to a distal end of a borescope and said camera attached to a proximal end of said borescope, and
- calculating the movement of the pattern or surface part relative to the distal end of the borescope based on the images captures by the camera.

In one embodiment of the invention the pattern comprises a plurality of markers, such as two or three markers. The pattern may comprise one or more disk shaped markers, such as two or three disk shape markers. Further, at least one marker may be an annulus. The pattern may thus comprise one, two or three markers, such as disks, enclosed by an annulus. Further, the position of a marker may be tracked by tracking the centre of gravity of the marker. And the determination of the movement in the image plane of the camera may be based on tracking of one or more markers, such as two or three markers, whereas the determination of the movement perpendicular to the image plane of the camera may based on tracking the annulus. The ellipticity and/or the diameter of the annulus in a camera image may further determined by calculating the centre line of the annulus.

When using a borescope for tracking the movement of an object in a location which is difficult to access and/or an object at least partly surrounded by a magnetic field, the object is located adjacent to a distal end of the borescope and a camera is attached to a proximal end of said borescope.

In the preferred embodiment of the invention, the pattern comprises at least one marker, such as two markers, three markers or more than three markers. The arrangement of said markers may at least partly define the pattern. Alternatively, the markers may be defined as fix points in the pattern, i.e. particular points in the pattern that are "fixed" and possibly easy to recognise and identify in a series of images. The pattern may be applied to the object or the pattern may be a natural pattern visible on the object. The pattern can also be a combination of an applied pattern and a natural occurring pattern on the object. The pattern may be applied directly to the surface of the object, such as drawings on the surface of the object, such as drawings on the skin of the patient in case of patient monitoring. The pattern may also be defined by markers fixed to the objects, such as markers screwed into bony structures of a patient and/or markers provided by scars and/or tattoos on a patient.

A natural pattern on the object may be a natural pattern in the skin of a patient, i.e. known from fingerprint reading. The pattern and/or the markers may for example be identified by texture recognition. The natural pattern may also be defined by anatomical structures and/or contours of a patient. Such patterns may be enhanced by illuminating the surface of the object and contours and/or structures of a pattern may be enhanced if the pattern is illuminated with light which is directed at the pattern at an angle different from the optical line of sight between camera and pattern. Preferably an angle which is between 10 and 80 degrees, such as between a 20 and 70 degrees, such as between a 30 and 60 degrees, such as between a 40 and 50 degrees.

Contours and structures may be further enhanced by using suitable light sources, such as a split lamp and/or a diffuse laser beam.

By applying a borescope, the sensitivity of known optical motion tracking technologies can be increased by orders of magnitude because the use of the borescope is the equivalent of having the camera close to the pattern because the focus point of the camera can by means of the optical relay system of the borescope be brought into close proximity of the pattern. And by having the camera at the other end of the borescope interference between the electronics in the camera and the electromagnetic field, that often surrounds the object of interest, is prevented. Further, the borescope allows access to locations that are hard to reach without requiring direct line of sight.

The motion tracking disclosed herein is described with a single camera approach where a pattern on the object / patient is tracked or a surface part of the object / patient is tracked. However, the present invention is not limited to a single borescope. More than one borescope may be provided. If more than one borescope is applied a stereovision system may be provided where 3D surface images of at least a part of the patient may be constructed, e.g. based on point clouds. A series of 3D surface images may be converted into patient movement monitoring. Thus, the 3D movement of the patient may be determined by monitoring a surface part of the patient, i.e. the 3D surface contour is monitored.

3D images may also be generated by means of tracking the 3D movement of a surface part using a single camera, e.g. utilizing a varying focus system. If a 3D surface contour is derived by imaging the surface part, the 3D movement of the object / patient may be tracked.

### Definitions

### Borescope (Wikipedia)

A borescope is an optical device consisting of a rigid or flexible tube with an eyepiece on one end, an objective lens on the other linked together by a relay optical system in between. The relay optical system can be surrounded by optical fibres for transmitting a light source and thereby illuminating a remote object. The tube is typically provided with a rigid or flexible protective outer sheath. An internal image formed by the objective lens is relayed to the eyepiece which typically magnifies the internal image and presents it directly to the viewer's eye via the eyepiece. The eyepiece may be fitted with a coupler lens to allow the borescope to be used with imaging devices such as a video or CCD camera.

Rigid borescopes are similar to a fiberscope but are not flexible. When in use inside the human body this type of device is referred to as an endoscope. Thus, the herein used term "borescope" may refer to a flexible or rigid borescope, fiberscope or endoscope.

Rigid borescopes generally provide a superior image at lower cost compared to a flexible borescope, but are however limited in that access to what is to be viewed must be in a straight line, whereas a flexible borescope can be used to access cavities which are around one or more bends.

The field of view of a rigid borescope can be in the direction of the tube, i.e. the borescope is imaging objects located in front of the distal end of the tube. However, the field of view may be vastly increased by providing an angled mirror at the distal end of the tube. If the mirror is provided at an angle of approx. 45 degree the field of view of the borescope will then be approx. perpendicular to the horizontal axis of the tube.

Flexible borescopes may suffer from pixilation and pixel cross talk due to necessary use of fibre image guides in the relay optical system. Image quality varies widely among different models of flexible borescopes depending on the number of fibres and construction used in the fibre image guide. For flexible borescopes, articulation mechanism components, range of articulation, field of view and angles of view of the objective lens are also important. Fibre content in the flexible relay is also critical to provide the highest possible resolution to the viewer. Minimal quantity is 10,000 pixels while the best images are obtained with higher numbers of fibres in the 15,000 to 22,000 range for the larger diameter borescopes.

The criteria for selecting a borescope are usually image clarity and access. For similar quality instruments, the largest rigid borescope that will fit the hole will give the best image. Relay optics in rigid borescopes can be of 3 basic types, Harold Hopkins rod lenses, achromatic doublets and gradient index rod lenses. For large diameter borescopes, the achromatic doublet relays work quite well, but as the diameter of the borescope tube gets smaller (less than about 4 millimetres) the Hopkins rod lens and gradient index rod lens designs provide superior images. For very small rigid borescopes, the gradient index lens relays are better.

### Electromagnetic field

The electromagnetic field can for example be the strong magnetic field provided by an MR based scanner, PET scanner, MRI-PET combination scanner, the X-ray field provided by a CT scanner, radiation as photons, particles e.g. electrons, betatrones, protons, neutrons and alpha particles from a radiotherapy apparatus and visible and invisible light as applied in for example laser therapy.

### Combination of clinical scanning techniques

Because PET imaging is most useful in combination with anatomical imaging, such as CT, modern PET scanners are now available with integrated high-end multi-detector-row CT scanners. Because the two scans can be performed in immediate sequence during the same session, with the patient not changing position between the two types of scans, the two sets of images are more precisely registered, so that areas of abnormality on the PET imaging can be more perfectly correlated with anatomy on the CT images. This is very useful in showing detailed views of moving organs or structures with higher anatomical variation, which is more common outside the brain.

Furthermore, PET scans are increasingly combined with MRI scans in so-called PET/MRI scanners. Needles to say, these combination scans further limit the allowable patient movement during scans.

### Patient

The term "patient" may be an individual human, mammal and/or animal, dead or alive. However, patient movement is often quite limited if the patient is dead.

### Detailed description of the invention

### Marker plate

A marker plate for use in an optical motion tracking application comprises a pattern on at least one side of the plate.

In one embodiment the pattern comprises three primary markers, the positions of the three primary markers forming a triangle, such as an equilateral triangle or an isosceles triangle. Distribution of the three primary markers in a triangular shape may facilitate determination of 3D motion of the marker plate. If for example the primary markers are distributed in a straight line the centre of mass of the three markers does not change during a rotation around that line, whereby this particular 3D motion in undetectable.

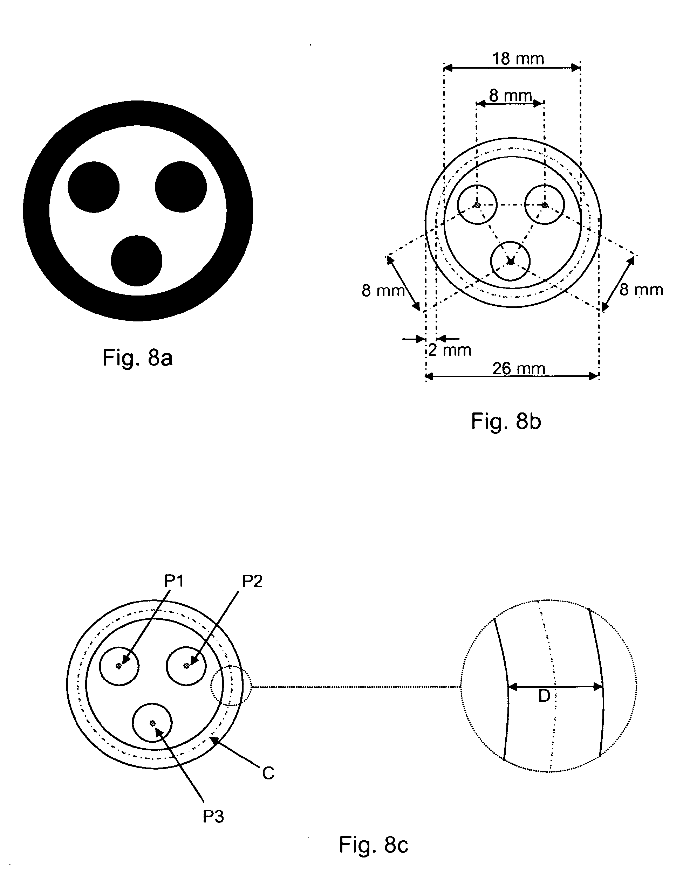

In one embodiment the pattern comprises one or more disk shaped markers, such as two or three disk shaped markers. Further, at least one marker may be an annulus. The pattern may thus comprise one, two or three markers, such as disks, enclosed by an annulus. See e.g. fig. 8 for an example of three disks enclosed by an annulus.

The marker plate may be flexible. The marker plate may for example be a piece of paper or adhesive tape or a sticker. The pattern may be pre-printed on the marker plate. The pattern be drawn by hand because only three markers are necessary for assessing the 3D motion of the marker plate, at least to a first order. I.e. in case of monitoring patient motion a sticker with at least three pre-printed or hand drawn markers may be applied to the skin of the patient. The sticker may be disposable, single-use and/or multiple use.

When using a centre of mass approach for determining 3D motion at least three markers are necessary when optically assessing the 3D motion of the marker plate, i.e. the three primary markers. However, the marker plate may further comprise a plurality of secondary markers. The secondary markers may facilitate the ability to correct for optical distortion in the optical system that images the marker plate in an imaging device. "Optical distortion" may also be termed just "distortion". The primary and secondary markers are not necessarily different. Three of the secondary markers may be selected as primary markers. However, processing images to determine the 3D motion of the marker plate can be less complicated when it is straightforward to identify and distinguish at least three of the markers and selecting these as primary markers. Thus, the area and/or the shape of the primary markers are different from the area and/or the shape of the secondary markers. Preferably the areas of the primary markers are larger than the areas of the secondary markers.

Correcting for optical distortion of the images is more straightforward and/or more precise if the location of the markers is predefined and well-known. Thus, least a part of the markers on the marker plate are positioned in a predefined geometrical pattern. One solution could be that at least a part of the primary and/or secondary markers are arranged in a pattern in accordance with a regular tessellation of a plane, such as arranged in the corners of a square tiling, a triangular tiling or a hexagonal tiling.

The necessary and/or possible number of secondary markers on the plate depends on the size of the plate, the size of the markers, the optics involved in imaging the marker plate, the resolution of the camera and/or the necessary number of markers to correct for optical distortion, etc. Thus, the number of secondary markers is between 1 and 500, such as between 5 and 300, such as between 10 and 100, such as between 15 and 80, such as between 20 and 70, such as between 25 and 60, such as between 30 and 50.

Similarly with the size of the marker plate which is a balancing between one or more of the following parameters: the size of the markers / the pattern, the optics involved in imaging the marker plate, the resolution of the camera, manageability, comfort for the patient, etc. Thus, the area of the marker plate may be below 10000 mm², such as below 5000 mm², such as below 4000 mm², such as below 3000 mm², such as below 2000 mm², such as below 1000 mm², such as below 800 mm², such as below 500 mm², such as below 400 mm², such as below 300 mm², such as below 200 mm², such as below 100 mm², such as below 80 mm², such as below 60 mm², such as below 40 mm², such as below 20 mm², such as below 10 mm², such as below 8 mm², such as below 6 mm², such as below 4 mm², such as below 2 mm², such as below 1 mm².

If the marker plate is applied in connection with optical motion tracking inside a multi-tesla strong magnetic field in the bore of a MRI scanner it is of extreme importance that the marker plate is non-magnetic. Thus, the marker plate is primarily manufactured in a non-magnetic material, such as plastics like thermosets. Preferably, the marker plate is substantially rigid.

During optical motion tracking the marker plate may be illuminated. This illumination might increase the temperature of the marker plate and possibly cause thermal expansion (or thermal reduction) of the marker plate and thereby perturb the calculation of 3D movement. Another source of heating may come from the patient's skin, where temperature changes may occur spontaneously or as a result of radiofrequency absorption. Thus, the marker plate should preferably primarily be manufactured in a material with a substantially low linear thermal expansion coefficient, such as a linear thermal expansion coefficient below 1000·10⁻⁶/°C, such as below 100·10⁻⁶/°C, such as below 50·10⁻⁶/°C, such as below 20·10⁻⁶/°C, such as below 10·10⁻⁶/°C, such as below 8·10⁻⁶/°C, such as below 6·10⁻⁶/°C, such as below 5·10⁻⁶/°C, such as below 4·10⁻⁶/°C, such as below 3·10⁻⁶/°C, such as below 2·10⁻⁶/°C, such as below 1·10⁻⁶/°C, such as between 2·10⁻⁶/C and 4·10⁻⁶/°C.

Suitable materials for a rigid marker plate could be selected from the group of thermosetting polymers, such as phenol-formaldehyde resins like Bakelite, aminoplastics, polyester fibreglass systems, vulcanized rubber, urea-formaldehyde foam, melamine resin, epoxy resins, fibre reinforced plastics, polyimides, PaperStone. A thermosetting polymer is polymer material that irreversibly cures. The phenol-formaldehyde Bakelit (a phenolic plastic) is also known as Isolit®, Carta, Pertinax®, Etronax and Etronit. Fillers like straw, carbon black, paper, sawdust, cotton, cellulose may be added to the phenolic resin. Thermoset materials may be liquid or malleable prior to curing and designed to be molded into their final form.

In optical motion tracking it is important that the pattern can be recognized on the marker plate, preferably optically recognized. Not necessarily recognized by looking directly at the marker plate with the human eye, but the applied image processing must be able to distinguish the pattern from the plate. In one embodiment the reflectance of the surface of the pattern is different than the reflectance of the surface of the marker plate itself, e.g. the reflectance of the surface of the pattern is higher or lower than the reflectance of the surface of the marker plate itself whereby the pattern will appear brighter respectively darker in an image of the marker plate.

Thus for example at least a part of the surface of the pattern may be coated with a layer with a reflectance that is different than the reflectance of the surface of the marker plate itself. And equivalently the surface of the marker plate may be coated with a layer with a reflectance that is different than the reflectance of the surface of the pattern.

Further, the surface of the pattern or the marker plate respectively may be provided in a colour/material that reflects light in a predefined wavelength interval whereas the surface of the marker plate itself or the pattern respectively is provided in a colour that substantially absorbs light in said wavelength interval.

The reflectance of light from the pattern can be higher than the reflectance of light from the surface of the marker plate itself for a predefined wavelength interval, such as more than 2 times higher, such as more than 3 times higher, such as more than 4 times higher, such as more than 6 times higher, such as more than 8 times higher, such as more than 10 times higher, such as more than 20 times higher, such as more than 50 times higher, such as more than 100 times higher, such as more than 500 times higher, such as more than 1000 times higher, such as more than 10000 times higher, such as more than 100000 times higher, such as more than 1000000 times higher.

This abovementioned predefined wavelength interval may be between 300 and 1200 nm, such as between 360 nm and 480 nm, such as between 380 nm and 450 nm, such as between 395 nm and 435 nm, such as between 410 nm and 430 nm, such as between 415 nm and 425 nm, such as between 400 nm and 700 nm, such as between 700 nm and 1200 nm, such as between 750 nm and 1100 nm, such as between 800 nm and 1000 nm, such as between 850 nm and 950 nm. The advantage of using light around 400 nm is that this short wavelength light is "colder" than longer wavelength light and the resulting heat increase of the marker plate is thereby less significant. However, using short wavelength light in the vicinity of the human eye might pose a risk. Thus, in these situations it might be more advantageous to use longer wavelength light, which is less harmful to the human eye, maybe even light above the visible spectrum. Longer wavelength light is also more applicable for transport in single-mode optical fibres, because the spectral loss decrease with increasing wavelength up until approx. 1600 nm for silica based optical fibres.

The marker plate can be at least partly manufactured by injection molding, extrusion molding and/or compression molding. Use of thermosetting plastics for the marker plate makes a molding manufacturing process possible.

The markers can be at least partly manufactured by means of abrasive blasting.

It may be important for the correct deduction of the 3D motion that the markers are distributed in the correct pattern. Thus, the markers may at least partly be burned into the marker plate by means of a laser as a part of the manufacturing process of the marker plate.

The surface of the markers may be at least partly below, level with or above the surface of the marker plate. E.g. the markers may be provided as depressions / recesses / dents / indentations / bores in the marker plate. In another embodiment the markers are provided as at least partly filled depressions / recesses / dents / indentations / bores in the marker plate. In yet another embodiment pattern and/or the markers are provided as studs / knobs / buttons on the marker plate, i.e. equivalent to a Lego brick.

The marker plate may comprise an adhesive layer, e.g. for attachment of the marker plate to the skin of an animal or human. The adhesive layer is preferably located on the side of the plate opposite the pattern. The purpose could be to make the marker plate suitable for attachment to one or more anatomical parts of a patient, such as the nose, forehead, skull, chest, leg, knee, arm, hand, foot, cheek, eyelid, ear, back, neck, chin, and/or the like. Preferably the marker plate further comprises a protective removable film on one or both sides of the plate. E.g. a protective film to be removed from an adhesive layer when fixing the marker plate on a patient. A protective film may also be applied to the pattern side of the plate to protect the markers from filth and dust and the like.

The marker plate may be disposable, such as a one-time use product to be discarded after use.

### The system

To be able to recognise the pattern in an image of the marker plate a contrast difference between the pattern and the adjacent surroundings may be necessary. As mentioned previously there are different means and methods for physically and optically enhancing the pattern on the marker plate. But some means of illumination may be required to facilitate recognition of the pattern. The background illumination in or around the scanner may suffer as illumination, however in order to provide a good resolution of the measurements a dedicated light source may be necessary.

The preferred embodiment of the invention therefore comprises a light source for illuminating at least a part of the pattern or the surface part with a probe light. With the provision of the borescope the most obvious illumination solution is to direct a probe light towards the pattern by means of the borescope, i.e. preferably a probe light is transmitted from the proximal end of the borescope to the distal end of the borescope thereby illuminating the pattern. A standard borescope comprises an optical relay system surrounded by optical fibres to transmit a probe light. Thus, the borescope solves the illumination limitations with known optical movement tracking techniques by literally moving the probe light in close proximity of the target (i.e. the pattern) but still avoiding having a light source inside an electromagnetic field in a limited scanning volume.

Commercially available light sources exist for commercially available borescopes. They are typically white light sources based on halogen or xenon lamps providing a powerful white light suitable for endoscopic video applications. The light is transmitted from the light source to the borescope by means of a guide cable with is typically a fibre bundle. However, illuminating the pattern with white light might result in unwanted reflections from the marker plate. A solution to that could be an optical filter. Thus the probe light can be provided by wavelength filtering light from a light source, such as a broadband light source, such as a white light source.

In yet another embodiment the reflected light from the marker plate is filtered. An optical filter may be provided somewhere between the light path from the marker plate to the camera, e.g. immediately in front of the camera. Filtering may also be provided in the image processing.

Illuminating a marker plate with a probe light is equivalent to directing an energy field towards the marker plate. Part of this energy will be transferred to the marker plate as heat possibly causing a temperature change of the marker plate that might result in a change of size of the plate due to thermal expansion / reduction. This effect can be reduced by applying a probe light with "cold" light, i.e. relatively low wavelength light. Thus, in one embodiment the probe light has a wavelength between 360 nm and 480 nm, such as between 380 nm and 450 nm, such as between 395 nm and 435 nm, such as between 410 nm and 430 nm, such as between 415 nm and 425 nm. Around 420 nm the light is still visible and relatively harmless to the human eye.

MRI is often applied when monitoring functions and special areas of the human brain. However, if the probe light is visible to the patients it may "disturb" the MRI scannings because the visible probe light might stimulate the wrong areas of the brain. Thus, in another embodiment the probe light has a wavelength above 750 nm, such as above 775 nm, such as above 800 nm, such as above 850 nm, such as above 900 nm, such as above 1000 nm. By applying probe light with a wavelength outside the visible spectrum it is ensured that e.g. MRI scanning is not adversely affected by a visible probe light. Light with a wavelength below the visible spectrum, i.e. below approx. 400 nm, can also be provided as probe light. However, UV probe light might pose a risk to the patient, especially if the probe light is applied in proximity of the head and eyes of the patient. Light with a wavelength above the visible spectrum may also be provided as probe light, such as probe light with a wavelength between 0,7 and 1,4 µm, such as between 1,4 and 3 µm, such as between 3 and 8 µm. Such long wavelength infrared light may be suitable to enhance contrast differences in the pattern, such as the contrast difference between the markers and the plate.

The pattern may reflect the probe light whereas the marker plate itself substantially absorbs the probe light.

A borescope comprises a tube accommodating an optical relay system. This tube may be rigid and may furthermore also be substantially straight. A substantially straight borescope provides the highest optical resolution and a low optical distortion. And even though a straight rigid borescope may be more unmanageable it may fit well inside the elongate scanner bore of a clinical scanner. However, the invention is not limited to a straight rigid borescope. A borescope with a flexible tube, such as a fibrescope, can also be used. To avoid interference with an electromagnetic field the tube of the borescope can for example be made of stainless steel or some plastic or ceramic material.

By applying the borescope in the herein described motion tracking application the distance between the camera and the object being tracked is substantially free to choose without compromising the resolution. Thus, in various embodiments of the invention the length of the borescope is between 0.2 and 10 meters, such as between 0.5 and 5 meters, such as between 0.75 and 3 meters, such as between 1 and 2 meters, such as more than 2 meters.

The electromagnetic field can for example be the strong magnetic field provided by an MR based scanner, the X-ray field provided by a CT scanner, the X-ray field provided by a CT scanner, radiation as photons, particles e.g. electrons, betatrones, protons, neutrons and alpha particles from a radiotherapy apparatus and visible and invisible light from e.g. laser therapy.

Thus, in one embodiment of the invention the object is a patient or an anatomical part of a patient and said patient is at least partly located in the bore / scanning volume of a clinical scanner, such as a MRI, fMRI, PET, CT, PET-CT, or PET-MRI scanner. The borescope is then preferably attached to the scanner and the marker plate is preferably attached to the anatomical part of interest, for example on the forehead, the nose or the chest of the patient. The surface part may be a part of the anatomical part of interest. The motion tracking system according to the invention may be an integral part of, incorporated, built-in, an add-on, supplementary and/or affixed to the clinical scanner. Thus the distal end of the borescope is preferably located adjacent to the patient and the proximal end of the borescope is preferably located outside the scanner bore.

In functional magnetic resonance imaging, for example, changes in the properties of blood in brain areas activated while subjects are performing tasks causes small signal changes (on the order of a few percent) that can be detected with MR. However, these small signal changes may easily be obscured by signal changes of similar or even greater size that occur during unintentional patient movements.

In addition to MRI, other types of scans require multiple repeated exposures, separated in time, of an entire (not slices) object (such as an organ, limb, hand or foot), such as angiograms and dynamic contrast enhanced studies, in which a dye or contrast agent is injected into a blood vessel and then scans separated in time are taken to determine how and where the dye spreads. These types of scans that detect motion inside a patient or other object over time ("digital angiography systems") can also have a long duration, and be subject to the problem of patient or object motion. The present invention may also be applicable in these cases.

In another embodiment at least a part of the pattern is luminous. This may be provided by illuminating the marker plate opposite the pattern.

### Motion tracking

There are different approaches to determine 3D motion using a single camera. One approach is to calculate the centre of gravity for a number of markers for each frame / image and tracking the motion of these centres of gravity. The term "centre of gravity" may also be termed "centroid" or "centre of mass". The centre of gravity of an object may be seen as the average of all points of the object, weighted by the local density or specific weight, respectively. Standard routines for calculating the centre of gravity of an object are available, e.g. the Matlab "centroid" function.

To be able to calculate the six degrees of freedom in 3D motion using the centre of gravity approach at least three distinguishable markers are necessary. These markers can be attached directly on the object, e.g. at the forehead or the chest of the patient. However, as previously indicated the preferred embodiment of the invention comprises a marker plate accommodating the markers, said marker plate being attached to the object.

Thus, in one embodiment of the invention the image processing means comprises means for calculating a centre of gravity for each of at least three markers for each camera frame, and means for calculating the change in position of the centres of gravity for each two consecutive frames, thereby determining the spatial movement of the object.

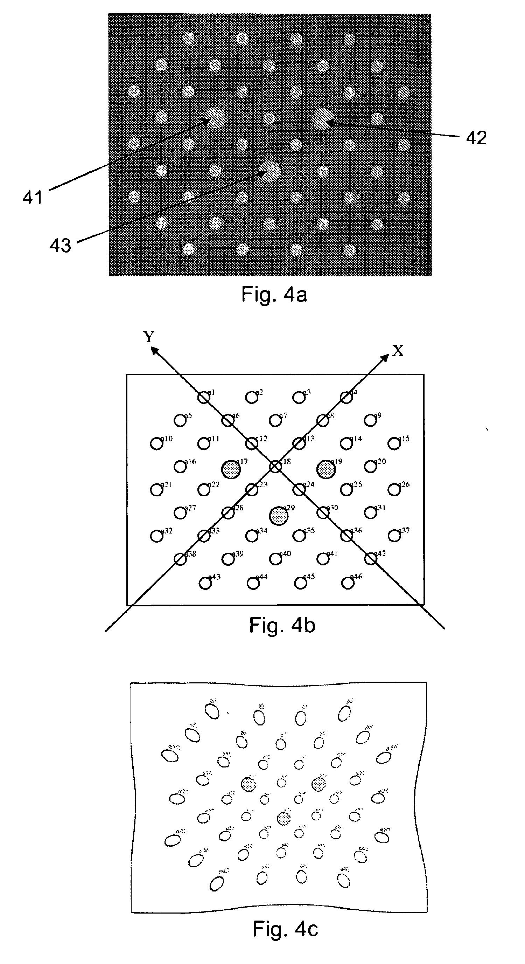

In the image processing a fixed (x,y)-coordinate system may be applied to each image of the marker plate, which is natural because each image comprises a number of pixels. An example of how to incorporate an (x,y)-coordinate system in the image of a marker plate is illustrated schematically in fig. 4b. The pattern of the marker plate is preferably predetermined, e.g. in terms of the distance between markers. By knowing the absolute geometry of the pattern a distance-to-pixel conversion factor can be calculated, e.g. by determining the distances in pixels between all three markers and comparing to the actual known distances in millimetres. The millimetre-to-pixel conversion factor is preferably determined for the first image (the reference image) in a series of images.

The calculation of the centre of gravity of each of the three markers can therefore provide an (x,y) coordinate for each centre of gravity (CoG) for each marker for each frame. The movement of these CoG coordinates can thereby be tracked for consecutive frames. If for example the x-coordinate of the CoG coordinates of all three markers increases by the same amount for two different frames whereas the corresponding y-coordinate is constant, it implies a movement of the marker plate in the x-direction in the time span between those frames. Knowing the CoG (x,y)-coordinates also provides for determining the distance between the markers and thereby the z-coordinate may also be determined. If for example the distance between all CoG coordinates of the three markers varies for two different frames, it implies that distance between the marker plate and the borescope has changed in the time span between the two frames. When the borescope gets closer to the marker plate the markers will seem to be wider apart. Thus, (x,y,z) coordinates may be determined and thereby 3D motion of the marker plate can de established.

If the calculated distances between the three markers in the reference image are not equal it may imply a tilt of the marker plate in relation to the image plane of the camera, the image plane of the camera is not parallel with the plane defined by the surface of the marker plate. This tilt may be determined by the difference in the calculated distances and can provide for a tilt conversion factor to be applied to the analysis of the subsequent images.

When optical elements are involved in transmitting the image of the marker plate to the camera, there will be a risk for optical distortion. Optical distortion can be minimized by using a borescope with a rigid tube, however not necessarily eliminating the effects of optical distortion. One embodiment of the invention therefore comprises means for calculating and correcting for the optical distortion in the optics of the borescope and/or the camera based on knowledge of the geometrical pattern of the markers. The optical distortion can for example be calculated by using one of the camera frames as a reference, preferably the first frame in a tracking series is the reference frame.

As indicated above only three markers are necessary to determine 3D motion of an object. However, by applying a larger predefined geometrical pattern to the marker plate may provide the means to calculate the optical distortion. E.g. straight lines on the marker plate may be imaged as curved lines in the camera due to the optical distortion of the optical elements imaging the marker plate in the camera. And distances between markers on the marker plate that should be equal or at least have a constant relation might change or vary in the images due to optical distortion. A crude illustration of optical distortion is shown in fig. 4c where a pincushion distortion wherein lines that do not go through the centre of the image are bowed inwards, towards the centre of the image. By determining the optical distortion of the reference image the optical distortion may be accounted for in the analysis of the rest of the images.

Distortion will typically be minimum in the centre of an image and it is therefore preferred that the primary markers are located near the centre of the marker plate. Further, the marker plate is preferably positioned in the centre of the camera frame.

The Achilles heel in the centre of gravity approach may be the determination of the z-component, because small variations in the z-axis may be undetectable in the camera plane and below the resolution of the system. Thus, a further embodiment of the invention comprises means for providing a local illuminated spot in the pattern. The shape of the spot may be circular, ovoid, square, rectangular, rhombus, hexagonal, or cross hair. Multiple spots may also be provided. The illuminated spot(s) may be provided by means of a light source directed at the object, such as a laser providing a laser dot on the surface of the object or on the marker plate, preferably a dot in the pattern. The light source can be fixed to the borescope, but this is not necessary. However, the light source is preferably fixed in relation to the borescope such that the borescope - and thereby the camera - and the light source cannot move in relation to each other.

Preferably the angle between the light source and the line of sight of the camera is different from zero, such as between 20 and 85 degrees, such as between 30 and 60 degrees, such as between 30 and 50 degrees.

The illuminated spot functions as an additional marker in the pattern. And because the spot is luminous it is easily recognizable in the image analysis. By having the illuminated spot fixed in relation to the borescope, movement of the spot in the image implies a change in z-position, i.e. a change in the distance between the borescope and the pattern. The (x,y)-coordinates can still be determined by observing and tracking the pattern, e.g. as described in the centre of gravity approach above. But by providing the illuminated spot the z-position is more easily calculated and consequently the various constraints on the system, e.g. the pattern, the optics, the camera, the marker plate, may be relaxed.

Another approach for determination of the z-component may be to provide at least one marker with the shape of a circle or an ellipse. Any movement in the z-axis will change the diameter / circumference of the circle / ellipse imaged in the camera, and tilt in the x-y plane around the z-axis will change the ellipticity of the circle / ellipse imaged in the camera compared to a reference. By knowing the exact size of the corresponding markers and knowing the pixel to distance conversion factor the z-component may be deduced. Instead of using a circle / ellipse an annulus (e.g. circular or elliptic) may be used and a circle / ellipse may be inscribed from e.g. the centre line of the annulus. An annulus will provide more pixels to define the marker in the image processing, thereby providing an increased signal to noise ratio.

### Drawings

The invention will now be described in detail with reference to the drawings in which
- Fig. 1: is a cross sectional illustration of a patient located in MRI scanner with a borescope attached to the scanner,
- Figs. 2a-e: show pictures of different examples of head coils for MRI scanners,
- Figs. 3a-c: show pictures of a filter and the corresponding filter holder,
- Fig. 4a: is a picture of a marker plate,
- Fig. 4b: shows an illustration of the marker plate in fig. 4a with embedded coordinate axes,
- Fig. 4c: shows an illustration of the marker plate in fig. 4a in case of optical distortion of the optics of the borescope and/or the camera,
- Fig. 5: is a schematic illustration of the luminous spot principle,
- Fig. 6a-d: show illustrations of a marker plate with a luminous spot,
- Fig. 7: shows an illustration of the triangle formed by the camera, the light source and the spot in the luminous spot principle,
- Fig. 8a-c: show a marker plate, and
- Fig. 9a-c: show data verifying the tracking system.

### Detailed description of the drawings

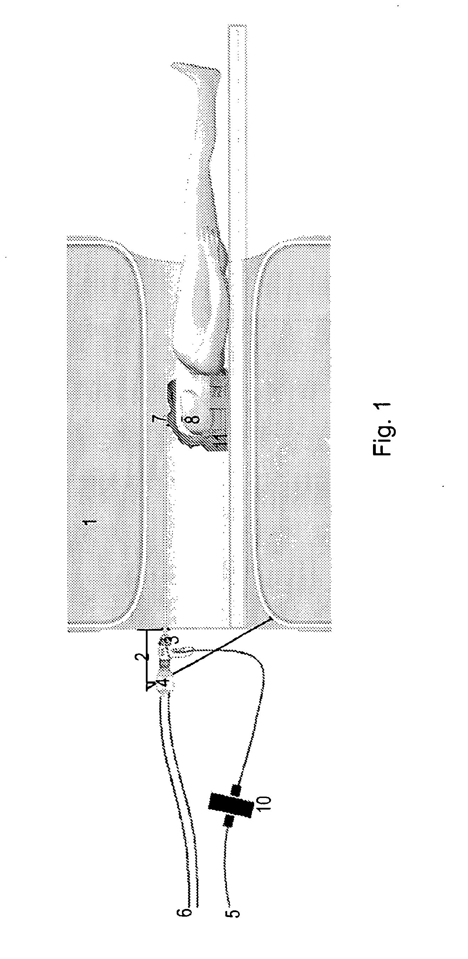

Fig. 1 shows a cut-through cross sectional side view illustration of an MRI scanner 1 with a patient located inside the scanner bore, in this example for examination of the brain. The powerful magnetic field generated by the scanner 1 aligns the magnetic moments of protons inside with the direction of the field. By varying the magnetic field the change in alignment of the protons creates a signal which can be detected, in this case detected by a head coil 11 that surrounds the head of the patient. In fig. 1 the head coil 11 is shown in cut-through to illustrate the head of the patient and the marker plate 8.

A borescope 3 with a rigid elongate tube is attached to the scanner by means of the rigid suspension attachment 2. A camera 4 is attached the proximal end of the borescope 3 which is substantially outside of the magnetic field of the scanner. The distal end 7 of the borescope is well inside the scanner bore just above the head coil 11 surrounding the head of the patient. A marker plate 8 is attached to the forehead of the patient and the distal end 7 of the borescope accommodates a reflective mirror allowing the borescope to look down on the head of the patient, whereby the marker plate 8, which may be virtual, is imaged in the camera 4. The connection wires 6 are the power to and the signal from the camera 4. Light from an external light source (not shown here) is transmitted to the borescope by means of the optical fibre based cable 5. An optical relay system incorporated in the borescope transmits the light further on to the distal end 7 of the borescope where it is shone on the marker plate 8 as probe light. An optical filter 10, such as an UV filter, is incorporated in the cable 5 to select a limited range of wavelengths from the light source.

The proximal end of the borescope 3 is mounted and fixed on scanner 1 by means of the suspension attachment 2. The distal end 7 of the borescope is mounted on and fixed to the head coil 11 and the head coil is mounted on and fixed to the scanner. Thus, when tracking the movement of the patient relative to the distal end 7 of the borescope 3 it will therefore also be a tracking of patient movement relative to the scanner 1.

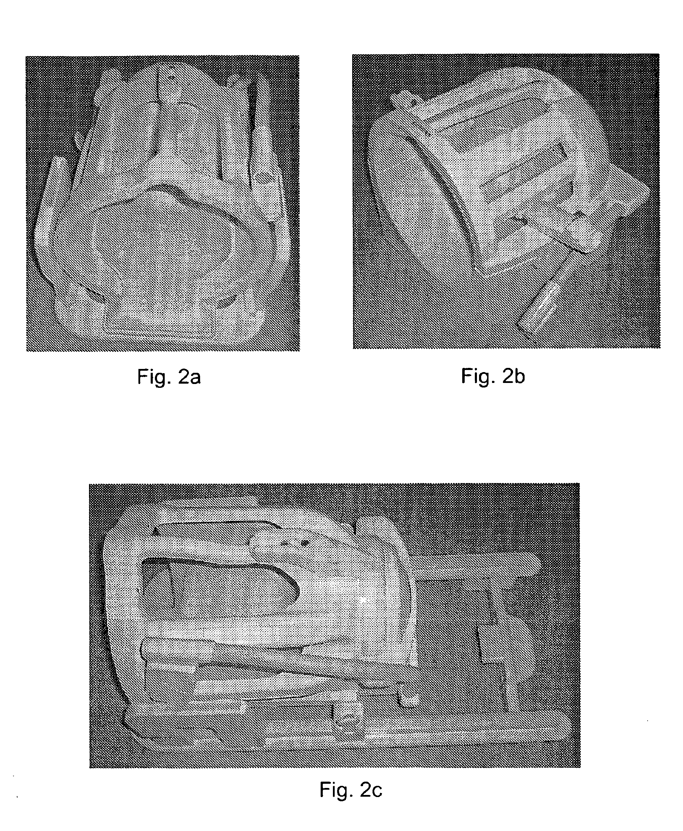

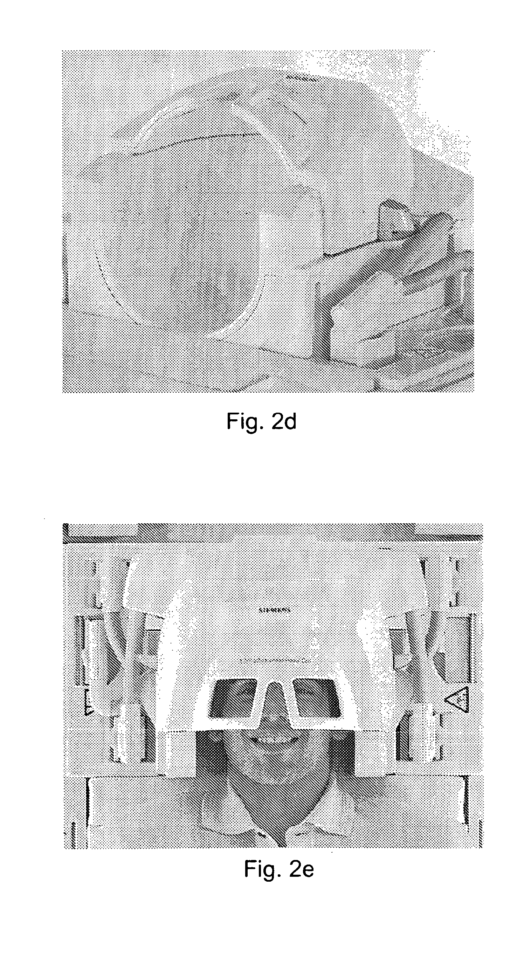

Figs. 2a, 2b and 2c show pictures of different head coils for MRI scanners viewed from different directions. Fig. 2d shows a picture of the most recent head coil for a Siemens MRI scanner with fig. 2e showing how this head coil surrounds the head of a person. The limited space and the presence of a strong electromagnetic field in the scanner bore makes access to the patient difficult during scanning. This access is even more restricted by the presence of the head coil, or another detector, which is an indispensable part of a MRI scanner, however severely limiting the access to the anatomical part of interest, i.e. the anatomical part being scanned. As seen from figs. 2d and 2e the most recent head coil even further limits the free space around the head and restricts the field of view to the patient. However, a borescope with a 90 degrees viewing direction as disclosed herein provides an excellent direct line of sight to a patient with the head inside the head coil.

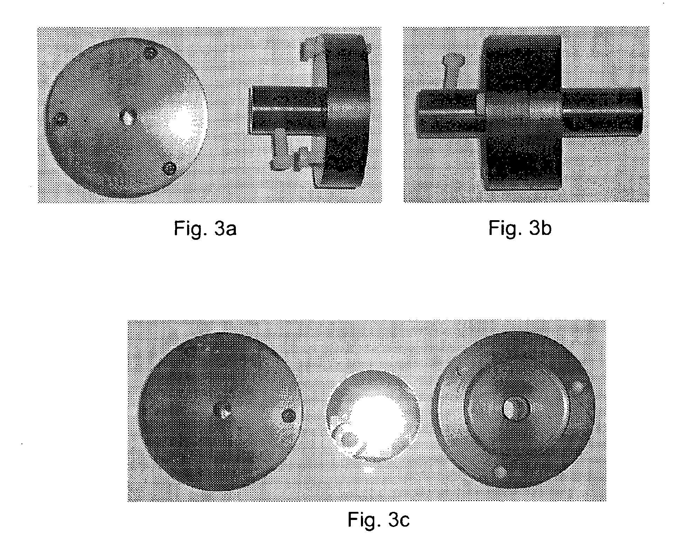

A white light source was employed as the external light source. However, the broad spectrum light provided too much reflection from the marker plate and the markers can thereby be difficult to distinguish from the marker plate. A solution was employed where a dichroic coated glass filter was fixed in a holder 10 and inserted in the light path of the cable 5. The filter holder comprises two separable parts as seen in fig. 3a. The parts have been assembled in fig. 3b with the filter in between. The filter and the two holder parts are seen in fig. 3c with the filter in the middle. A central aperture for the light can be seen in both parts of the holder. In one embodiment a deep purple filter was used providing a probe light in the 420 nm region.

Fig. 4a shows an image of an exemplary embodiment of a marker plate with a pattern defined by a plurality of markers. In this case forty-six markers where the three big markers 41, 42, 43 (i.e. the primary markers) are clearly distinguishable from the other forty-three markers (the secondary markers). The marker plate itself is substantially black for better absorption of the light whereas the markers are coloured light to better reflect the incoming probe light. This difference in absorption and reflection between the marker plate and the surroundings on the marker plate provides for a sufficient contrast difference to be able to optically recognize the markers. In the centre of gravity approach at least three markers are necessary to be able to deduce 3D motion of the marker plate. In this case the three primary markers 41, 42, 43 are used for 3D tracking, whereas the secondary markers may be practical when determining a possible image distortion provided by the optics. I.e. when the image of the pattern is distorted compared to the actual appearance of the pattern. This error consists in the different parts of the pattern being reproduced with different magnifications. Fig. 4b illustrate the geometrical pattern of an exemplary marker plate with embedded coordinate axes, whereas Fig. 4c shows an exaggerated illustration of the same marker plate in case of image distortion provided by the optics of the borescope and/or the camera, in this case a pincushion-shaped image distortion.

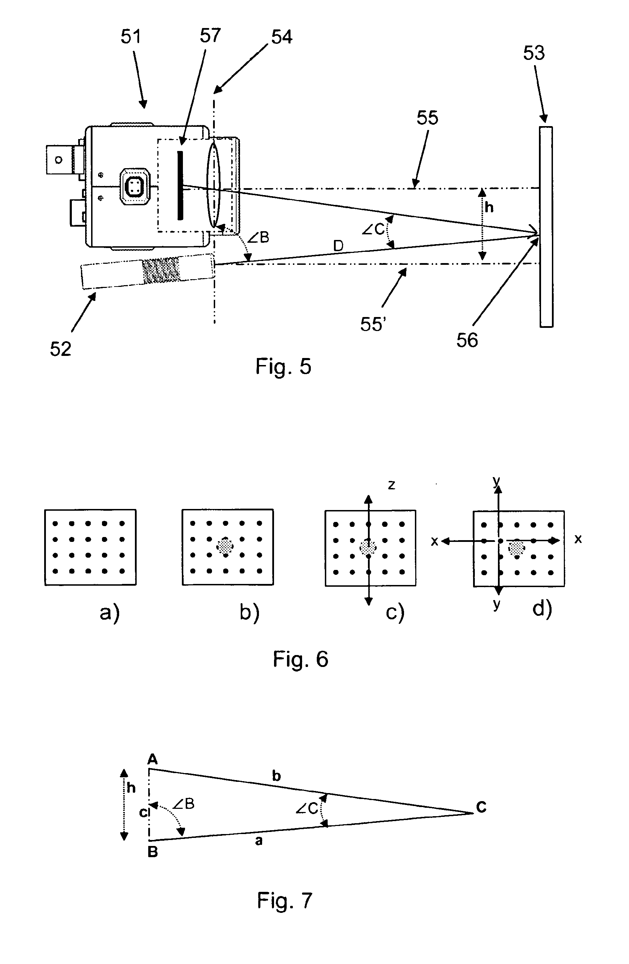

Fig. 5 is an illustration of the luminous spot principle which may be provided for a better and more easy determination of the z-component in the movement of the pattern. The pattern is incorporated on the surface of a marker plate 53 seen from one side, i.e. the pattern is not visible in fig. 5. The camera 51 and a laser are fixed in relation to each other with the laser 52 aligned with the image plane of the camera 51 along the line 54. This is an illustration of the principle. In this invention the camera will be located at one end of a borescope with the pattern fixed to the object at the opposite end of the borescope. But as previously explained the borescope translates the image plane of the camera to the distal end of the borescope. Thus, in practise the light source 52 as illustrated in fig. 5 will be fixed in relation to and near the distal end of the borescope.

The laser 52 produces a spot 56 on the surface of the marker plate 53 and this spot is detected at a specific position in the sensor-array 57 in the camera 51. The constant h is the distance between the laser 52 and the camera. When the distance between the line 54 (defined by the camera 51 and laser 52 positions) and the marker plate 53 varies the detected position of the spot 56 in the sensor array 57 is also varying. In an (x,y,z)-coordinate system with the (x,y)-axes defined by the marker plate surface, a variation of the distance between the line 54 and the marker plate 53 translates into a variation along the z-axis. Thus, when determining the spatial movement, the (x,y)-coordinates can be determined by monitoring the movement of the pattern along those two axes, whereas the z-coordinate can be determined by monitoring the position of the spot 56 in the sensor array 57.

This is further illustrated in Fig. 6 where 6a shows an illustration of an image of a marker plate with a squared tessellation pattern of markers. In 6b in the laser spot is illustrated as the big spot in the middle. In fig. 6c it is illustrated that when the distance between the marker plate and the laser + camera is varied the laser spot will move up and down in the image of the marker plate, i.e. the detected position of the spot in the sensor array moves up and down. In fig. 6d it is illustrated that the (x,y) coordinates of the marker plate movement can be monitored by just following the movement of the pattern. The spot will be easily distinguishable from the pattern on the pattern. The necessary field of view of the camera 51 can be minimized, because in case of regular pattern only the spot and part of the pattern needs to be imaged. I.e. the field of view can be limited to a small area around the luminous spot.

The luminous spot principle may also be provided without the marker plate, i.e. when the pattern is a natural pattern identified at the surface of the object and/or a pattern drawn on the surface of the object. The luminous spot is then directed directly at the surface of the object, e.g. directly at the skin of a patient.

Fig. 7 shows an illustration of the triangle formed by the positions A: the camera, B: the laser and C: the laser spot on the marker plate 53. The distance h between A and B is constant and the angle B is also constant. The angle C will vary when the distance from B to C varies. By simple geometric considerations the variation (in pixels) in spot position in the sensor array 57 can be converted to a variation in distance (e.g. in millimetres) between the camera 51 and the marker plate 56.

Fig. 8 shows an exemplary embodiment of a pattern defined by a plurality of markers. In this case the markers are three disks and an annulus. The disks are arranged in a triangle and enclosed by the annulus. Fig. 8a is an enlarged version of the pattern. In the actual physical realisation of the pattern as part of a marker plate it may be made quite small as illustrated in the example in fig. 8b with an outer diameter of the annulus of 26 mm, an inner diameter of 18 mm and a width of the ring of the annulus of 4 mm. The disks are spaced with 8 mm between the centres and located in an equilateral triangle.

As previously indicated it may be difficult to determine the spatial movement in the direction perpendicular to the image plane of the camera (i.e. along the z-axis where the x-y plane is the image plane of the camera) when a single camera is used. Especially if the spatial tracking is solely based on tracking the centre of gravity of the markers of the pattern. But by adding a circle, an ellipse or preferably an annulus to the pattern the movement in the z-axis may be based on tracking and/or characterising the circle, the ellipse or the annulus as imaged in the camera. In particular the ellipticity and/or the diameter and/or the circumference. The ellipticity may be used to provide the tilt of the pattern in the image plane of the camera. The diameter and/or the circumference, in particular the maximum diameter, of the annulus may be used to provide the relative movement in the z-axis. E.g. if the maximum diameter of the annulus is decreasing between two images, the pattern is moving away from the camera. The diameter of the annulus may be the inner diameter (as indicated by 18 mm in fig. 8b), the outer diameter (as indicated by 26 mm in fig. 8b) or more preferably the diameter of the centre line C of the annulus. The centre line C is indicated by a dotted line in figs. 8b and 8c and is defined as the line connecting the midpoints between the inner and outer ring around the edge of the annulus.

When the pattern as depicted in fig. 8 is used for spatial movement tracking, the centre of gravity P1, P2, P3 is determined for each disk in the camera images. Further, the centre line C of the annulus is determined in the camera images. If the centre line C appears as an ellipse due to tilt of the pattern the maximum diameter of the centre line C corresponds to the major axis of the ellipse. The centre line C determined from the annulus is merely a circle. But it provides a better signal to noise ratio compared to a normal circle depicted in the pattern, because much more pixels are involved in determining the centre line C compared to a circle. Further, only one marker would suffer to determine the movement in the image plane but more markers increases the signal to noise ratio and thereby the statistical precision of the movement tracking.

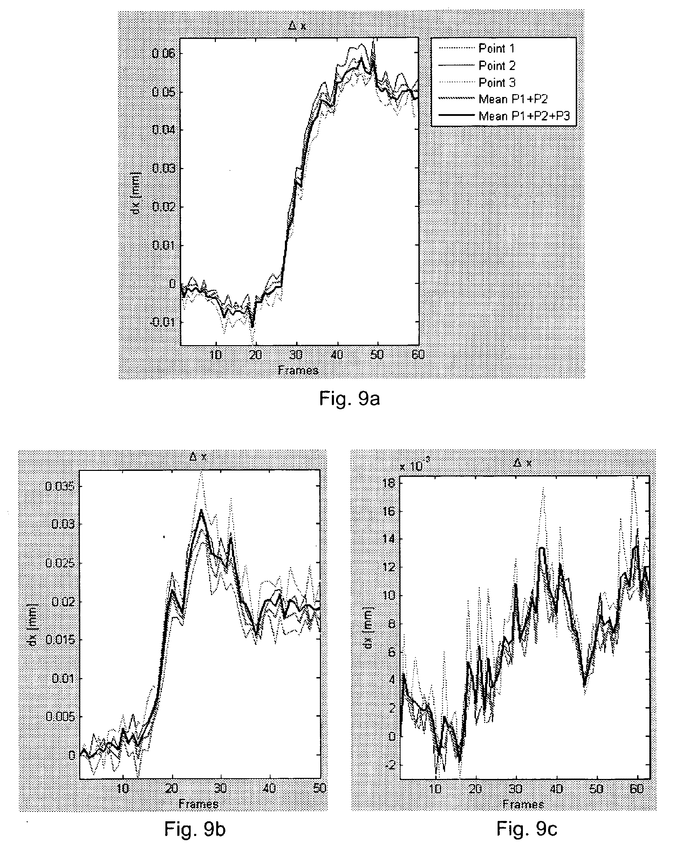

Fig. 9 show three graphs illustrating measurements that was performed to verify the motion tracking abilities of the system. A marker plate with a pattern as shown in fig. 8a was placed in a micrometer calliper adjacent to the distal end of a borescope. A camera located at the proximal end of the borescope was continuously recording images of the pattern while the micrometer calliper was adjusted. The three graphs in fig. 9 show the time in camera frames along the first axis and changes in the x-position along the second axis. At time = 0 the x-position is 0. When the micrometer calliper is adjusted the pattern changes position which is instantly observed by the motion tracking system and visualized in the graphs. In fig. 9a the x-position is changed 0.05 mm, in fig. 9b the x-position is changed 0.02 mm and in fig. 9c the x-position is changed only 0.01 mm. There is some overshoot because the micrometer calliper adjustment is performed manually by hand. The different curves in each graph show the changes in markers P1, P2 and P3. The thick black curve in the graph shows a mean of the change of the three markers. The conclusion is that the motion tracking system according to the present invention and realized in an exemplary embodiment here can track movements of an object located in a strong electromagnetic field below 0.01 mm in real-time.

### Applications

A borescope can be used for tracking the movement of an object in a hardly accessible location and/or an object at least partly surrounded by a magnetic field, i.e. utilizing any of the herein described system features and/or marker plate features.

The invention may be applied in various applications. A further embodiment of the invention relates to a clinical scanner for imaging at least a part of an anatomical part of a patient incorporating a system according to any of the herein described features for tracking the spatial movement of said anatomical part of said patient. A clinical scanner may be any of the following scanner types: MRI, fMRI, PET, CT, PET-CT, and PET-MRI.

Tracking of the spatial movement of a patient during scanning may provide knowledge of whether the acquired scan data is acceptable or should be discarded due to too much patient motion. However, this invention may provide for real-time spatial motion tracking and thereby adaptive scanning is possible by incorporating the present invention in a scanning method. Thus, a further embodiment of the invention relates to a method for adaptively scanning at least a part of an anatomical part of a patient in a clinical scanner, said method comprising the steps of:
- initiating the determination of the spatial movement of said anatomical part according to any of the herein described methods, thereby producing data representing the spatial movement of said anatomical part,
- initiating clinical scanning of said patient thereby producing clinical scan data, and
- adaptive and/or real-time correction of the scanning data based on the spatial movement data.

Yet another embodiment of the invention relates to an apparatus for providing image guided therapy to at least a part of an anatomical part of a patient incorporating a system and/or a marker plate according to any of the herein described features for tracking the spatial movement of said anatomical part of said patient. The image guided therapy may be any of the following therapy types: stereotactic radiotherapy, stereotactic radio surgery, ultrasound health therapy and laser therapy.

By having the means and the methods for tracking the patient motion during a therapeutic treatment it can assured that the therapy is directed at the correct location. E.g. the therapy may be halted, permanently or temporarily, as soon as the patient moves outside a predetermined interval. A further embodiment of the invention relates to a method for adaptive image guided therapeutic treatment of at least a part of an anatomical part of a patient, said method comprising the steps of:
- initiating the determination of the spatial movement of said anatomical part according to any of the herein described methods, thereby producing data representing the spatial movement of said anatomical part,
- initiating image guided therapy of said patient, and
- adaptive and/or real-time correction of the image guided therapy based on the spatial movement data.

## Claims

1. An optical motion tracking system for determining the movement of an object at least partly located in a volume of difficult access and/or at least partly located in an electromagnetic field, said system comprising:
- a borescope for imaging a pattern on the object or a surface part of the object in a camera, said pattern or surface part located adjacent to a distal end of the borescope and said camera attached to a proximal end of said borescope, and
- image processing means for calculating the movement of said pattern or surface part relative to the distal end of the borescope based on a plurality of frames / images captured by the camera.

2. The system according to any of the preceding claims, wherein the pattern is defined by at least one marker, at least two markers, at least three markers or more than three markers.

3. The system according to claim 2, wherein one or more markers are disk shaped and/or wherein at least one marker is an annulus.

4. The system according to any of the preceding claims, wherein the pattern comprises one, two or three markers, such as disks, enclosed by an annulus.

5. The system according to any of the preceding claims, further comprising a marker plate accommodating the pattern, said marker plate being attached to the object.

6. The system according to any of the preceding claims, wherein a probe light is transmitted from the proximal end of the borescope to the distal end of the borescope thereby illuminating the pattern or the surface part, and/or wherein the field of view of the borescope is along the longitudinal direction of the borescope or along a direction substantially perpendicular to the longitudinal direction of the borescope.

7. The system according to any of the preceding claims, wherein the object is an individual or an anatomical part of an individual, said individual at least partly located in the bore of a clinical scanner, such as a MRI, fMRI, PET, CT, PET-CT, or PET-MRI scanner, the borescope being attached / fixed to said scanner.

8. A method for determining the spatial movement of an object at least partly located in a volume of difficult access and/or at least partly surrounded by an electromagnetic field, said method comprising the steps of:
- continuously imaging in a camera a pattern on the object or a surface part of the object, said pattern or surface part located adjacent to a distal end of a borescope and said camera attached to a proximal end of said borescope, and
- calculating the spatial movement of the pattern or surface part relative to the distal end of the borescope based on the images from the camera.

9. The method according to claim 8, wherein the pattern is located on at least one side of a marker plate, said marker plate being attached to the object.

10. A clinical scanner for imaging at least a part of an anatomical part of a patient comprising a motion tracking system according to any of the claims 1 to 7 for tracking the spatial movement of said anatomical part of said patient.

11. The clinical scanner according to claim 10, wherein the motion tracking system is incorporated, built-in, an add-on, supplementary and/or affixed to the clinical scanner and/or wherein the scanner is selected from the group of MRI, fMRI, PET, CT, PET-CT, and PET-MRI scanners.

12. An apparatus for providing image guided therapy to at least a part of an anatomical part of a patient comprising a motion tracking system according to any of the claims 1 to 7 for tracking the spatial movement of said anatomical part of said patient.

13. A method for adaptively scanning at least a part of an anatomical part of a patient in a clinical scanner, said method comprising the steps of:
• initiating the determination of the spatial movement of said anatomical part according to any of claims 8 to 9 thereby producing data representing the spatial movement of said anatomical part,
• initiating clinical scanning of said patient thereby producing clinical scan data, and
• adaptive and/or real-time correction of the scanning data based on the spatial movement data.

## Patentansprüche

1. System zur optischen Bewegungsverfolgung zum Bestimmen der Bewegung eines Objekts, das zumindest teilweise in einem schwer zugänglichen Raum angeordnet ist und/oder zumindest teilweise in einem elektromagnetischen Feld angeordnet ist, wobei das System umfasst:
- ein Boroskop zum Abbilden eines Musters auf dem Objekt oder einem Oberflächenteil des Objekts in einer Kamera, wobei das Muster oder der Oberflächenteil an einem fernen Ende des Boroskops angrenzend angeordnet sind und die Kamera an einem nahen Ende des Boroskops angebracht ist, und
- Bildverarbeitungsmittel zum Berechnen der Bewegung des Musters oder des Oberflächenteils bezüglich des fernen Endes des Boroskops auf Grundlage einer Mehrzahl von von der Kamera festgehaltenen Rahmen/Bildern.

2. System nach einem der vorstehenden Ansprüche, wobei das Muster durch mindestens eine Markierung, mindestens zwei Markierungen, mindestens drei Markierungen oder mehr als drei Markierungen definiert ist.

3. System nach Anspruch 2, wobei eine oder mehr Markierungen scheibenförmig sind und/oder wobei mindestens eine Markierung ein Kranz ist.

4. System nach einem der vorstehenden Ansprüche, wobei das Muster ein, zwei oder drei Markierungen wie beispielsweise Scheiben umfasst, die von einem Kranz eingeschlossen sind.

5. System nach einem der vorstehenden Ansprüche, ferner ein Markierungsschild umfassend, das das Muster beherbergt, wobei das Markierungsschild an dem Objekt angebracht ist.

6. System nach einem der vorstehenden Ansprüche, wobei ein Probenlicht von dem nahen Ende des Boroskops zu dem fernen Ende des Boroskops gesendet wird, wodurch das Muster oder der Oberflächenteil beleuchtet wird, und/oder wobei das Blickfeld des Boroskops entlang der Längsrichtung des Boroskops oder entlang einer Richtung, die im Wesentlichen senkrecht zu der Längsrichtung des Boroskops ist, verläuft.

7. System nach einem der vorstehenden Ansprüche, wobei das Objekt eine Person oder ein anatomischer Teil einer Person ist, wobei sich die Person zumindest teilweise in dem Hohlraum eines klinischen Scanners wie beispielsweise eines MRT-, fMRT-, PET-, CT-, PET-CT- oder PET-MRI-Scanners, wobei das Boroskop an dem Scanner angebracht/befestigt ist.

8. Verfahren zum Bestimmen der räumlichen Bewegung eines Objekts, das zumindest teilweise in einem schwer zugänglichen Raum angeordnet ist und/oder zumindest teilweise von einem elektromagnetischen Feld umgeben ist, wobei das Verfahren die folgenden Schritte umfasst:
- kontinuierliches Abbilden eines Musters an dem Objekt oder eines Oberflächenteils des Objekts in einer Kamera, wobei das Muster oder der Oberflächenteil an das ferne Ende des Boroskops angrenzend angeordnet ist und die Kamera an einem nahen Ende des Boroskops angebracht ist, und
- Berechnen der räumlichen Bewegung des Musters oder des Oberflächenteils bezüglich des fernen Endes des Boroskops auf Grundlage einer Mehrzahl von Bildern von der Kamera.

9. Verfahren nach Anspruch 8, wobei das Muster an mindestens einer Seite des Markierungsschildes angeordnet ist, wobei das Markierungsschild an dem Objekt angebracht ist.

10. Klinischer Scanner zum Abbilden zumindest eines Teils eines anatomischen Teils eines Patienten, umfassend ein System zur Bewegungsverfolgung nach einem der Ansprüche 1 bis 7 zum Verfolgen der räumlichen Bewegung des anatomischen Teils des Patienten.

11. Klinischer Scanner nach Anspruch 10, wobei das System zur Bewegungsverfolgung in den klinischen Scanner integriert ist, eingebaut ist, ihn erweitert, ihn ergänzt und/oder an ihm befestigt ist und/oder wobei der Scanner aus der Gruppe bestehend aus MRT-, fMRT-, PET-, CT-, PET-CT- und PET-MRI-Scannern ausgewählt ist.

12. Vorrichtung zum Bereitstellen bildgesteuerter Therapie für zumindest einen Teil eines anatomischen Teils eines Patienten, umfassend ein System zur Bewegungsverfolgung nach einem der Ansprüche 1 bis 7 zum Verfolgen der räumlichen Bewegung des anatomischen Teils des Patienten.

13. Verfahren zum adaptiven Scannen zumindest eines Teils eines Patienten in einem klinischen Scanner, wobei das Verfahren die folgenden Schritte umfasst:
• Initiieren des Bestimmens der räumlichen Bewegung des anatomischen Teils nach einem der Ansprüche 8 oder 9, wodurch Daten erstellt werden, die die räumliche Bewegung des anatomischen Teils repräsentieren,
• Initiieren des klinischen Scannens des Patienten, wodurch Daten zum klinischen Scan erstellt werden, und
• Adaptive und/oder Echtzeit-Korrektur der Scandaten auf Grundlage der Daten zur räumlichen Bewegung.

## Revendications

1. Système optique de capture de mouvement, conçu pour déterminer le déplacement d'un objet au moins partiellement disposé dans un volume d'accès difficile et/ou au moins partiellement disposé dans un champ électromagnétique, ledit système comprenant :
- un horoscope destiné à produire une image d'un motif sur l'objet ou sur une partie de la surface de l'objet dans une caméra, ledit motif ou ladite surface étant disposé(e) de manière adjacente à une extrémité distale de ledit horoscope et ladite caméra étant fixée à une extrémité proximale dudit boroscope, et
- des moyens de traitement d'image pour calculer le déplacement dudit motif ou de ladite partie de surface par rapport à l'extrémité distale du horoscope sur la base d'une pluralité de vues/images capturées par la caméra.

2. Système selon l'une quelconque des revendications précédentes, dans lequel le motif est défini par au moins un marqueur, au moins deux marqueurs, au moins trois marqueurs ou plus de trois marqueurs.

3. Système selon la revendication 2, dans lequel un ou plusieurs marqueurs est/sont en forme de disque et/ou dans lequel au moins un marqueur est un anneau.

4. Système selon l'une quelconque des revendications précédentes, dans lequel le motif comprend un, deux ou trois marqueurs tels que des disques, encerclés par l'anneau.

5. Système selon l'une quelconque des revendications précédentes, comprenant en outre une plaque de marquage accueillant le motif, ladite plaque de marquage étant fixée à l'objet.

6. Système selon l'une quelconque des revendications précédentes, dans lequel une lumière d'exploration est transmise de l'extrémité proximale du horoscope à l'extrémité distale du horoscope de manière à éclairer le motif ou la partie de surface, et/ou dans lequel le champ de vue du horoscope s'étend dans le sens longitudinal du horoscope ou le long d'une direction sensiblement perpendiculaire au sens longitudinal du boroscope.

7. Système selon l'une quelconque des revendications précédentes, dans lequel l'objet est une partie individuelle ou anatomique d'un individu, ledit individu étant au moins partiellement disposé dans le tunnel d'un dispositif de balayage clinique, tel qu'un dispositif IRM, IRMf, TEP, TDM, TEP-TDEM ou TEP-IRM, ledit boroscope étant monté/fixé audit dispositif de balayage.

8. Procédé de détermination du déplacement dans l'espace d'un objet au moins partiellement disposé dans un volume d'accès difficile et/ou au moins partiellement disposé dans un champ électromagnétique, ledit procédé comprenant les étapes consistant à :
- produire en continu dans une caméra des images d'un motif sur l'objet ou sur une partie de surface de l'objet, ledit motif ou ladite partie de surface étant disposé(e) de manière adjacente à une extrémité distale d'un boroscope et ladite caméra étant fixée à une extrémité proximale dudit boroscope, et
- calculer le déplacement dans l'espace du motif ou de la partie de surface par rapport à l'extrémité distale du boroscope sur la base des images provenant de la caméra.

9. Procédé selon la revendication 8, dans lequel le motif est disposé sur au moins un côté d'une plaque de marquage, ladite plaque de marquage étant fixée à l'objet.

10. Dispositif de balayage clinique conçu pour produire des images d'au moins une partie d'une partie anatomique d'un patient, comprenant un système de capture de mouvement selon l'une quelconque des revendications 1 à 7 pour capturer le déplacement dans l'espace de ladite partie anatomique dudit patient.

11. Dispositif de balayage clinique selon la revendication 10, dans lequel le dispositif de capture de mouvement est incorporé, intégré, rapporté, ajouté et/ou fixé au dispositif de balayage clinique et/ou dans lequel le dispositif de balayage est sélectionné parmi le groupe des dispositifs IRM, IRMf, TEP, TDM, TEP-TDEM ou TEP-IRM.

12. Appareil destiné à effectuer une thérapie guidée par image sur au moins une partie d'une partie anatomique d'un patient, comprenant un système de capture de mouvement selon l'une quelconque des revendications 1 à 7 pour capturer le déplacement dans l'espace de ladite partie anatomique dudit patient.

13. Procédé de balayage adaptatif d'au moins une partie d'une partie anatomique d'un patient, ledit procédé comprenant les étapes consistant à :
- initier la détermination du déplacement dans l'espace de ladite partie anatomique selon l'une quelconque des revendications 8 à 9 de manière à produire des données représentant le déplacement dans l'espace de ladite partie anatomique,
- initier le balayage clinique dudit patient de manière à produire des données de balayage clinique, et
- effectuer une correction adaptative et/ou en temps réel des données de balayage sur la base des données de déplacement dans l'espace.
